# EUROPEAN PATENT SPECIFICATION

(11) **EP 3 207 463 B1**
(45) Date of publication and mention of the grant of the patent: **02.04.2025**
(21) Application number: 15851181.6
(22) Date of filing: 16.10.2015
(51) Int. Cl.: G06F 17/00, G06Q 10/08

(54) **REMOTE FILLING, TRACKING AND VERIFICATION OF PHARMACY SUPPLY CONTAINERS**
ENTFERNTE BEFÜLLUNG, VERFOLGUNG UND ÜBERPRÜFUNG VON ARZNEIMITTELVERSORGUNGSBEHÄLTERN
REMPLISSAGE, SUIVI ET VÉRIFICATION À DISTANCE DE RÉCIPIENTS DE PRODUITS PHARMACEUTIQUES

(30) Priority: 16.10.2014 US 201462064911 P
(43) Date of publication of application: 23.08.2017
(73) Proprietor: Gsl Solutions Inc., Vancouver, WA (US)
(72) Inventor: LOUIE, Shelton, Vancouver, WA 98683 (US); INTILE, Joseph, Vancouver, WA 98683 (US); GARRETT, Stephen A., Vancouver, WA 98683 (US)
(74) Representative: Kalkoff & Partner Patentanwälte mbB
(86) International application number: PCT/US2015/056107
(87) International publication number: WO 2016/061559

(56) References cited:
- WO-A1-2006/084310
- WO-A1-2008/006203
- US-A- 5 771 657
- US-A1- 2003 050 731
- US-A1- 2005 049 746
- US-A1- 2009 321 470
- US-A1- 2010 268 548
- US-A1- 2011 146 835
- US-A1- 2011 146 835
- US-B2- 7 860 603

## Description

### BACKGROUND

Automated systems and methods for monitoring and tracking prescription orders, pharmacy supply containers, filled prescriptions and the like are known. Examples of these systems can be found in U.S. Pat. No. 8,479,988 to Louie, et al., and U.S. Pat. No. 7,747,477 to Louie et al.,.

Also, local retail pharmacies, healthcare facilities and the like are increasingly relying on remote, "central fill" pharmacies to fill prescription orders of customers and patients and ship the filled prescriptions to the local retail pharmacy or healthcare facility for dispensing to the customer or patient. An exemplar, remote "central fill" pharmacy system is shown in U.S. Pat. No. 7,448,544 to Louie et al..

In addition, automated prescription filling machines are known and in common use. These machines automatically count and dispense a desired medication into a container and apply a label containing customer identifying information. Some of these machines include automated verification systems, such as measuring the weight of the filled prescription and comparing it to a predetermined

weight and flagging an error if the detected weight differs from the predetermined weight. These systems can also include automated visual detection systems such as cameras or the like that can physically count the number of pills dispensed and the appearance of the pills and compare those detected values with predetermined criteria and flag any discrepancies detected. Exemplar automated filling system are shown in U.S. Pat. No. 8,275,481 to Rice et al., U.S. Patent Application Serial No. 13/473,267 to Luciano, et al., and U.S. Patent Application Serial No. 13/325,782 to Rhoads. These automated verification systems have proven themselves to be accurate and reliable.

In general, a licensed pharmacy technician or pharmacist is required to verify that a prescription for a customer has been properly filled. This verification usually includes verifying that the proper medication has been loaded into the correct locations within an automated filling machine, and that the proper amount of that medication has been placed in the container earmarked for the customer/patient, and that the customer's/patient's container is properly labeled. Labeling includes identifying information about the medication, quantity, how to take the medication contained therein and the customer/patient it is intended for.

In cases where a prescription order is filled at a remote facility, there may be several licensed pharmacy technicians and/or pharmacists along the filling flow process that perform the same verification steps for the same prescription and supply container orders. While such redundant verification is certainly beneficial in the rare case where verification along the flow process is erroneous, it needlessly increases the workload of the pharmacy technicians/pharmacists. Moreover, it requires more licensed pharmacy technicians & pharmacists to be involved in the filling and dispensing process than needed to accurately and consistently dispense the correct medications to patients and customers. Since the demand on licensed pharmacy technicians and pharmacists is continuing to increase, these redundant verification systems can compromise a pharmacy's ability to timely dispense medications to patients and customers.

Moreover, most medication has Unique Drug Identity ("UDI") information associated with it including its lot number, expiration date, pedigree code, National Drug Code ("NDC"), Drug Identification Number ("DIN") and the like. This information is provided by the manufacturer of the medication with the supply container. However, much of this information is lost for medication withdrawn from the supply containers.

US 2005/049746 A1, WO 2008/006203 A1, and US 2011/146835 A1 disclose devices, systems and methods for dispensing drugs.

### SUMMARY

Thus, despite the known systems for filling prescription orders of customers and patients and filling and loading supply containers containing medications into automated filling machines, there remains a need for a cost effective system that consistently and accurately verifies prescription orders and the contents of supply containers without needlessly requiring multiple verifications from different licensed pharmacy technicians or pharmacists.

Moreover, there remains a need for medication UDI information of a supply of medication to follow the filling flow through a system to each individual filled prescription order of a customer or patient. The present invention fulfills these and other needs.

The advantages and features of novelty characterizing aspects of the invention are pointed out with particularity in the appended claims. To gain an improved understanding of the advantages and features of novelty, however, reference may be made to the following descriptive matter and accompanying figures that describe and illustrate various configurations and concepts related to the invention.

### FIGURE DESCRIPTIONS

The foregoing Summary and the following Detailed Description will be better understood when read in conjunction with the accompanying figures.

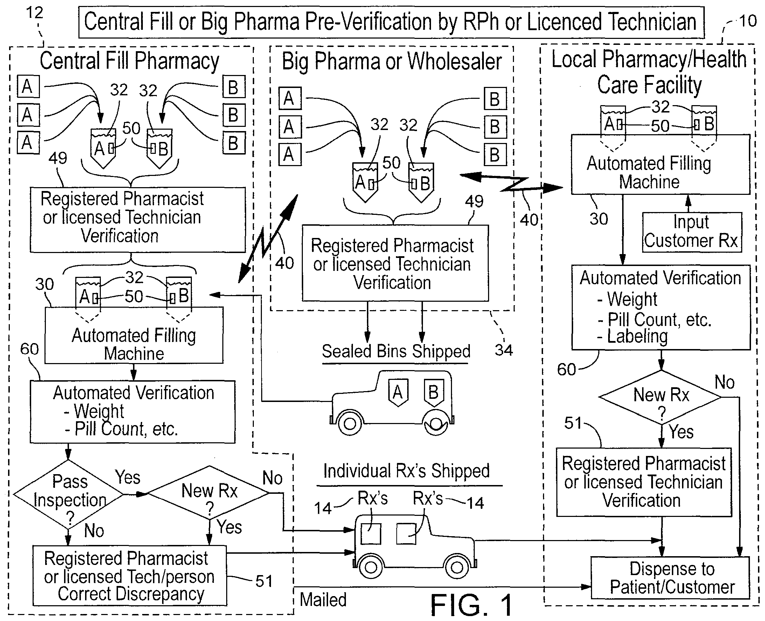

FIG. 1 is flow chart of an exemplar pharmacy filling system that uses a central fill pharmacy to fill the prescription order and a local pharmacy to dispense the prescription order to a customer or patient in accordance with an embodiment of the present invention. The automated filling machine is filled with medications contained in bins that have been filled and verified by a pharmacist or licensed pharmacy technician at a third party location, such as a wholesaler, a pharmaceutical manufacturer, or a central fill location.

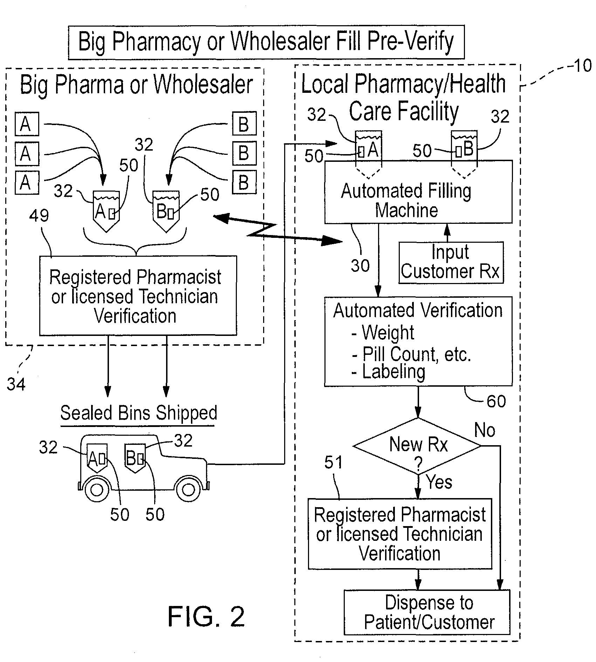

FIG. 2 is a flow chart of an exemplar pharmacy filling system that uses a local pharmacy or healthcare facility to fill the prescription orders by relying on a third party, such as a wholesaler or pharmaceutical manufacturer, to provide bins filled with supply medications that have been filed and verified by a pharmacist or licensed pharmacy technician at the third party location.

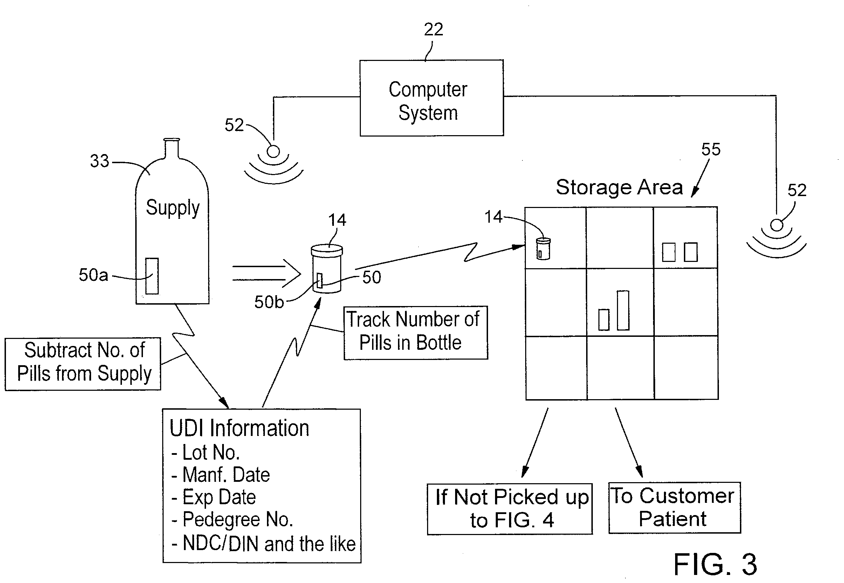

FIG. 3 is a schematic diagram of an exemplar filling of an individual prescription order of a customer from a stock supply of the prescribed medication showing the transfer from Unique Drug Identifier ("UDI") information about the medication in the stock supply container to a computer readable tag that travels with the individual prescription order in accordance with an embodiment of the present invention. In a disclosed embodiment, a computer system uses the tag to track, monitor and locate the individual prescription, correlate it with a customer, and maintain UDI information of the medication contained with the individual prescription.

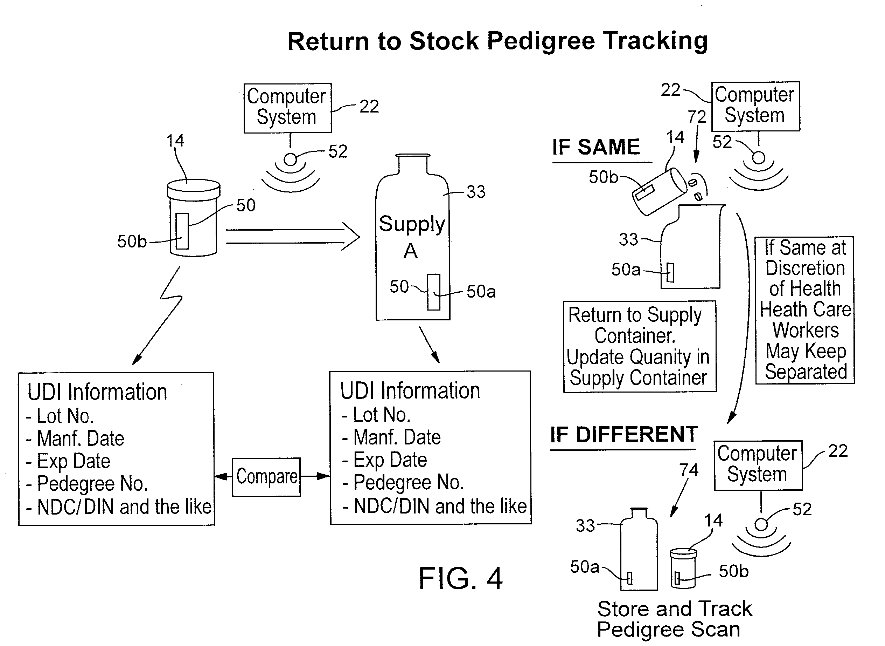

FIG. 4 is a schematic diagram of an exemplar "Return to Stock" comparison whereby the computer system compares the UDI of an individual prescription order that was not timely dispensed to a customer or patient to the UDI of a supply container and authorizes the return of the medication to the stock supply container only if the UDI's between them are identical.

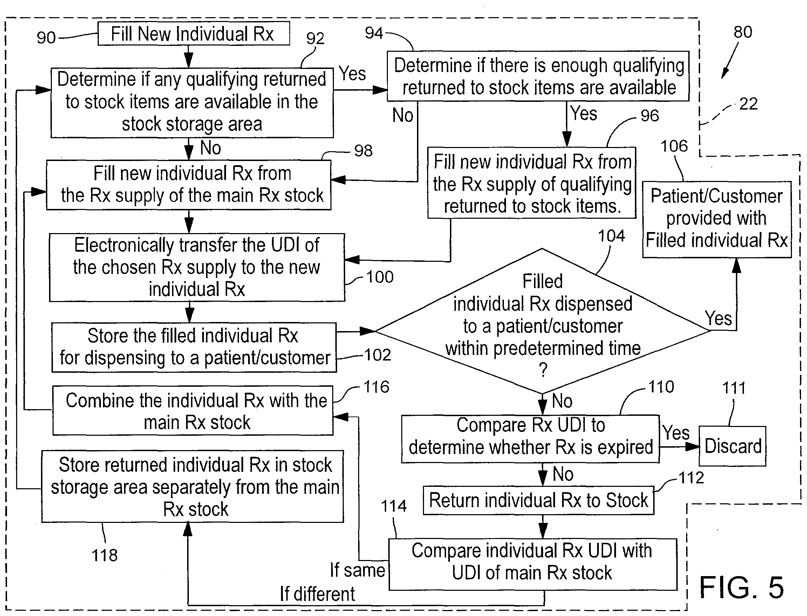

FIG. 5 is a flow chart of an exemplar prescription order filling system with a computer system monitored and verified "return to stock" feature that minimizes medication loss while preserving UDI integrity of the returned to stock medications.

### DETAILED DESCRIPTION

A pharmacy filling system is shown in FIGS. 1-5. A local pharmacy 10 or healthcare facility may use a central fill pharmacy 12 to assist with filling an individual prescription order 14 as shown in FIG. 1, or it may fill the prescription order 14 within the local or retail pharmacy 10 itself as shown in FIG. 2. FIGS. 3-5 show an exemplar prescription medication dispensing system 20 to a customer or patent with a computer system 22 monitored and verified "return to stock" feature that minimizes medication loss while preserving the Unique Drug Identifier ("UDI") of medication that is returned to stock. Each of these features is discussed in greater detail below.

### Automated Prescription Filling with System Tracking and Verification

Regarding FIGS. 1 & 2, in both embodiments, an automated filling machine or system 30 is used to fill the prescription orders 14. The automated filling system 30 is located at the central fill facility 12 in the embodiment of FIG. 1, and at the local pharmacy 10 or healthcare facility in the embodiment in FIG. 2. At either location, the automated filling system 30 is stocked with medication supply bins 32 that have been filled and verified by a pharmacist and/or licensed pharmacy worker 49 at a remote location 34 such as a wholesaler or the medication manufacturer. The local pharmacy 10 or healthcare facility is preferably in electronic or computer communication 40 with the remote location 34 and central fill facility 12, if applicable.

The supply bins 32 are sealed and tagged at the remote location 34, preferably with an electronic tag 50 such as a barcode, RF tag, RFID tag, GPS tag, or the like, that travels with each supply bin 32. The tag 50 includes identifying information about the medication contained within the supply bin 32 to which it is attached. Preferably, the tag 50 is in communication with a computer system 22 that includes one or more tag readers 52 that detect the presence of the tag to determine its location. The computer system 22 includes a database that may include detailed information about the medication contained within the supply bin 32 including its location as well as UDI information about the medication.

More preferably, the tag 50 is a radio-frequency identification tag ("RFID"), and includes Unique Drug Identity ("UDI") information regarding the medication contained within the bin 32. Such information can include the lot number, expiration date, drug name, drug strength, pedigree number, fill date, pharmacist/licensed pharmacy worker verification and identification, and the like for the medication contained in the bin.

The sealed bins 32 are transported to their respective automated filling system 30, which is either the central fill pharmacy 12 in the embodiment of FIG. 1, or the local pharmacy/health care facility 10 in the embodiment of FIG. 2. A local worker installs the bins 32 into the automated filling system 30 by breaking the seal. A local tracking system in communication with the computer system 22, such as a system disclosed in U.S. Patent No. 8,479,988 to Louie, et al. or the like, uses a tag reader 52 or the like to automatically read the information contained on the tag 50 that is operably secured to the bin 32. The computer system 22 automatically verifies that the correct bin 32 has been placed in the correct location within the automated filling system 30 without requiring a local licensed pharmacy worker or pharmacists to verify the contents of the bin 32 or its proper location within the automated filling machine 30.

Should the computer system 22 detect a discrepancy, such as the wrong supply bin 32 being installed in the wrong location, the system 22 can alert a worker to correct the discrepancy or call of a licensed pharmacy worker or pharmacist to intervene to correct the situation.

During filling of the prescription order 14, either at the central fill pharmacy 12 in FIG. 1 or at the local pharmacy 10 or healthcare facility of FIG. 2, the supply bins 32 and the prescription orders 14 are tagged with electronic machine readable tags 50 that monitor and track their location within the pharmacy and include information about the prescription orders and/or supply bin contents (e.g. UDI's) as needed.

The automated filling machine 30 may include standard automated filling verification systems 60 such as weight verification, label verification, pill count verification, video comparison of the pills to an image of the pill in a standard catalog of pills, and the like. These verification systems 60 verify that the automated filling machine 30 properly placed the correct medication and the correct amount of that medication into a container that has been properly labeled for a particular customer or patient. Should these systems detect a discrepancy, the system alerts a pharmacy worker of any discrepancies where that particular prescription order 14 is pulled from the system and manually inspected and corrected by a pharmacy worker before it is released to a customer or patient.

It can be appreciated; that with the foregoing systems, individual electronic tagging 50, tracking and monitoring of the supply bins 32 and the prescription orders 14, and automated verifications systems 60 downstream of the filling machines 30, a filled prescription order 14 for a refilled prescription may be dispensed to a customer with only one manual verification of the supply bins 32, by a healthcare worker, well upstream of the individual filling of the prescription 14. The downstream automated monitoring, testing, tracking and verification performed by the computer system 22 maintains the integrity of the supply bins 32 and the resulting filled prescription orders 14.

Of course, the filling system can include additional inspection stations along the filling path as needed to comply with local pharmacy dispensing regulations. For example, if a prescription order 14 is new, and calls for new prescription handling or patient counseling, a registered pharmacy worker and/or pharmacist 51 can be called in to verify that particular order and counsel the customer or patient. Refilled orders can pass this step saving licensed pharmacy worker's and pharmacist's time.

If desired, the pharmacist or licensed pharmacy worker 49 at a remote location 34 and/or the pharmacists or licensed pharmacy worker 51 at the local pharmacy 10 can each wear a tag 50 that is in communication with the computer system 22. This allows for the computer system 22 to monitor, track and document which workers performed which tasks related to both the supply bins 32 and individual prescription orders 14

### System Tracking and Verification of Unique Drug Identifier

Referring to FIGS. 3-5, the computer system 22 can monitor and track the Unique Drug Identifier ("UDI") information about the medication in each supply container 33 within the system, such as lot number, manufacturer date, expiration date, drug name, drug strength, pedigree number, National Drug Code ("NDC"), Drug Identification Number ("DIN") and the like. The supply container 33 may be from a previously packaged bin 32 or a separate container that arrived at the pharmacy by other methods. As shown in FIG. 3, this UDI information can be transferred to each individual prescription order 14 when medication from that supply container 33 is transferred to an individual prescription order 14.

For example, the supply container can include a first machine readable tag 50a that is readable by a tag reader 52 in communication with a computer system 22. A second machine readable tag 50b can be operably secured to the individual prescription order 14. The first tag 50a can include UDI information about the medication contained in the supply container 33 including the remaining volume or number of pills contained therein. When the individual prescription order 14 is filled and a portion of the contents of the supply container 33 are transferred to the container containing the individual prescription order 14, the computer system 22 can detect this activity and associate the individual prescription order 14 of that tagged individual prescription container to a customer or patent's prescription order. The computer system can transfer the resulting UDI information of the supply container 33 to the tag 50b associated with the individual prescription order 14. This UDI information from the supply container 33 travels with the individual prescription order 14 to a storage area 55 until that order is dispensed to a customer or patient.

### UDI Information Preserved When Medication is Returned to Stock

Referring to FIG. 4, should an individual prescription order 14 be returned to stock or the like, the system can track and store the UDI information about the returned medication, thereby allowing it to be re-dispensed without risk of it becoming expired or without knowing its exact pedigree. The computer system 22 can read the tag 50b associated with the returned prescription order 14 and the tag 50a associated with the supply container 33 and compare the UDI's of each. If key items of UDI's are identical, such as lot number, expiration date, NDC and pedigree number, the returned medication can be placed back into the supply container 33 without compromising the integrity of the supply container 33 as shown by arrow 72 in FIG. 4. The system can activate one or more transducers if the transfer of the returned medication is not authorized and alert a pharmacy worker if the UDI of the supply container 33 has been compromised.

Alternatively, if the UDI's between the returned medication and the supply container 33 do not match, the returned medication can remain within the storage area for use to fill a new prescription order as shown by arrow 74 in FIG. 4. The computer system 22 can alert a pharmacy worker whether to use medication from the returned prescription order or from the supply container.

Having described how the computer system 22 maintains and tracks UDI information from the supply containers 33 to the individual filled prescription orders 14. It can be appreciated that medication loss can be minimized by preserving UDI integrity of the returned to stock medications, and inadvertent dispensing of expired medications to customers and patents can be eliminated.

An exemplar individual prescription filling system 80 taking full advantage of maintaining medication UDI information integrity throughout the filling process is shown in FIG. 5. In step 90, a new individual prescription order is provided to the pharmacy. The pharmacy first determines if there are qualifying returned to stock medications available to fill the new prescription order (Step 92). If there is, the system next determines if there is enough of the returned to stock medication to fill the new prescription order (Step 94) and if there is the new prescription order is filled with from the returned to stock supply (Step 96). In cases where the supply of returned medication is greater than the amount dispensed to the new individual prescription order the computer system may update the volume or quantity of medication in the returned to stock supply as medication is dispensed to fill the new prescription order.

Alternatively, if there is none or not a sufficient amount of returned to stock medication to fill the new prescription order, the system directs a pharmacy worker or an automated fill system to fill the new prescription order from the main stock supply container of the prescribed medication (Step 98).

After the individual filled prescription order is filled either from an existing returned to stock supply or from the main stock supply, the computer system transfers the UDI information of the source supply to the computer readable tag associated with the individual prescription order (Step 100). More preferably, the computer system also monitors and tracks the volume or pill count in both the supply container and the volume or number of pills placed in the container of the individual prescription. For example, it consults the database associated with that customer/patient and determines the number of pills prescribed and deducts that amount from the selected supply container and adds them to the filled individual prescription order.

The filled individual prescription order is then stored for dispensing to a customer or patient (Step 102), and a clock is initiated to track how long the individual prescription order remains in the storage area. Preferably, the individual prescription is stored in an area that is in communication with the computer system 22 to automatically monitor, detect, and log user access.

As shown in Step 104, if the individual prescription order is dispensed to a patient or customer within a predetermined time, further tracking of the UDI information and location tracking of the customer's order can stop (step 106). The UDI and other information can be stored in a database for further reference or documentation as needed.

Alternatively, if the individual medication is not dispensed to a patient or customer OR within a predetermined time, the medication within the individual prescription can be returned to stock (step 112). However, because the UDI information of the supply container is preserved and transferred to each individual prescription filled, should a medication contained within a filled prescription order waiting for pick-up expire before it is picked-up by a customer or dispensed to a patient (step 110), the system can flag the discrepancy to a pharmacy worker, such as by activating a transducer on the tag associated with that prescription order, who can correct the situation before the customer seeks to pick up the medication or before it is dispensed to a patient (step 111). The computer system 22 can further prevent dispensing of an expired medication to a patient or customer by taking additional security steps such as calling for a pharmacist override before it will unlock or locate the prescription order for the pharmacy worker, or by activating an audible warning alarm or the like.

If desired, the computer system can also update the UDI information of the individual prescription order 14 and the supply container 33 with a user entered or pharmacy pre-selected configurable date. Preferably, this configurable date is earlier than the expiration date of the medication contained in the respective individual prescription order 14 and supply container 33, and it is selected to give a patient or customer a reasonable time to use the medication before it will expire. The previously described flagging and warning system for expired medications could also be activated upon reaching this configurable date, thereby preventing the dispensing of medications that do not have a reasonable time to be used before they expire.

Also, the electronic tracking of pedigree information of medications in the supply container also allows the pharmacy to dispense medications that are closer to their expiration dates first, and avoid dispensing medications that are too close to their expiration dates to be meaningfully used by a customer or patient before they expire. Using the oldest inventory first and avoiding disposing of expired or nearly expired medications with this system also saves money for the pharmacy.

The computer system first compares the UDI information of each individual prescription order with the UDI information of the supply container for that particular medication (step 114). If the UDI information is the same, the medication contained within the individual supply container can be returned to the supply container (step 116). The pill count or volume of medication in the individual prescription order returned to the supply container can be automatically added by the computer system to the UDI information of the supply container. Alternatively, if the UDI information between the individual prescription order differs from the UDI information of the supply container, the returned to stock medication can be stored and tracked for further use (step 118) as previously described. Of course, the worker may choose to store separately even if the UDI's match to save time.

One skilled in the relevant art will recognize that numerous variations and modifications may be made to the configurations described above. For example, if desired in step 94, if there is not a sufficient amount of medication in a returned to stock supply of medication, a pharmacy may fill only a partial amount of an individual prescription order from the returned to stock supply. If it does this, it can pull the remaining supply from the main stock of the medication supply. If it combines medication from these two sources of supply medication into one individual container, the UDI information of that container is compromised. Accordingly, the computer system will track this individual prescription order to ensure that it is not returned to stock for reuse. Alternatively, the pharmacy may provide two individual containers, each with an individual machine readable tag and each containing the UDI information of the supply source from which it was filled. In such case, the medication contained within each individual container may be returned to stock as previously described while maintaining UDI integrity of all the medications.

Also, the storage area 55 (FIG. 3) for filled prescription orders 14 needs to be spaced apart from the storage area of the supply containers. In such case, should a filled individual prescription order be flagged for being returned to stock and stored in the same area where the supply of that medication is also stored, the "return to stock" function can be purely electronic, whereby the computer system simply reclassifies that that individual prescription order as being returned to stock medication without that item actually being moved. Of course, any customer identifying labeling information would still need to be removed before the returned item could be dispensed to a new customer.

## Claims

1. A pharmacy prescription order filling system comprising:
an automated prescription filling machine (30) located at a first location that dispenses individual prescription orders from at least one bin (32) containing a stock supply of medication therein;
the at least one bin (32) operably secured to a computer readable tag (50) in communication with a computer system (22), said computer readable tag (50) identifying the bin (32) to the computer system (22);
the computer system (22) including a database containing at least one unique drug identifier (UDI) about the stock supply of medication contained in the bin (32);
the bin (32) filled with the stock supply of medication and sealed at a second location remote from the first location;
the bin (32) transported from the second location to the first location and inserted into the automated prescription filling machine with the computer readable tag (50) in communication with the computer system (22);
the computer system (22) using a tag reader (52) to automatically read the information contained on the computer readable tag (50) and automatically associating the UDI with the bin and verifying proper placement of the bin (32) in the automated prescription filling machine (30);
a supply container (33) including a first machine readable tag (50a) that is readable by the tag reader (52) in communication with the computer system (22), the first tag (50a) including UDI information about medication contained in the supply container (33) including the remaining volume or number of pills contained within the supply container (33), the supply container (33) being from the bin (32) or a separate container;
the automated prescription filling machine (30) filling a prescription order container (14), the prescription order container (14) having a second machine readable tag (50b) secured thereto; and
the computer system (22), when medication from the supply container (33) is transferred to the prescription order container (14), detecting the transferring and, responsive to detecting the transferring, transferring at least a portion of the UDI information from the first tag (50a) to the second tag (50b),
wherein the UDI information travels with the prescription order container (14) to a storage area (55) until the prescription order container (14) is dispensed to a customer or patient;
the computer system (22), when the prescription order container (14) is returned, reading the second tag (50b) associated with the returned prescription order container (14) and the first tag (50a) of the of the supply container (33) and comparing UDI's of the first and second tags;
when the UDIs are identical, including lot number and expiration date and national drug code, allowing the placement of the contents of the returned prescription order container (14) back into supply container (33); and
when the UDIs do not match, allowing the returned prescription order container (14) to remain in the storage area (55) for use to fill a new prescription order.

2. The pharmacy prescription order filling system of claim 1, further including an automated verification system (60) operably secured to the automated prescription filling machine (30) to automatically inspect the prescription order prior to being dispensed.

3. The pharmacy prescription order filling system of claim 1, wherein the filled prescription order is dispensed to a customer or patient at the first location.

4. The pharmacy prescription order filling system of claim 3, wherein the first location is a local pharmacy and the second location is selected from the group consisting of a medication wholesaler, an offsite centralized replenishment facility designed specifically to tag and refill bins and a medication manufacturer.

5. The pharmacy prescription order filling system of claim 3, wherein the local pharmacy (10) is a retail pharmacy.

6. The pharmacy prescription order filling system of claim 3, wherein the local pharmacy (10) is a pharmacy within a healthcare facility.

7. The pharmacy prescription order filling system of claim 1, wherein the filled prescription order is dispensed to a customer or patient at a third location.

8. The pharmacy prescription order filling system of claim 7, wherein the first location is a central fill pharmacy (12), the second location is a medication wholesaler, an offsite centralized replenishment facility designed specifically to tag and refill bins, or a medication manufacturer, and the third location is a retail pharmacy, a pharmacy within a healthcare facility, or a customer's address.

9. The pharmacy prescription order filling system of claim 8, wherein the third location is a customer's address and the filled prescription order is shipped from the first location to the customer's address.

10. The pharmacy prescription order filling system of claim 1, wherein each of the first and second machine readable tags is a Radio Frequency Identification ("RFID") tag, and further including a first tag reader (52) in communication with the computer system (22) positioned at the first location and a second tag reader in communication with the computer system positioned at the second location.

11. The pharmacy prescription order filling system of claim 10, further including a plurality of the first tag readers spaced apart from each other at the first location.

12. The pharmacy prescription order filling system of claim 1, wherein
the bin (32) being sealed at the second location and inspected by a certified pharmacy worker who verifies its contents at the second location,
the system further including a third machine readable tag operably secured to the certified pharmacy worker, and the computer system (22) in communication with the third tag to track and monitor the location of the certified pharmacy worker relative to the bin during filling and verification of the bin.

13. The pharmacy prescription order filling system of claim 1, wherein the seal on the bin is a hermetic seal.

14. The pharmacy prescription order filling system of claim 1, further including a transducer in communication with the computer system (22) that activates to alert a pharmacy worker when the computer system detects a predetermined discrepancy associated with filling the individual prescription.

15. The pharmacy prescription order filling system of claim 14 wherein the predetermined discrepancy is selected from the group consisting of improper medication dispensed from the automated prescription filling machine (30), improper supply bin positioned in the automated prescription filling machine and improper filled prescription removed from a storage area.

16. The pharmacy prescription order filling system of one of claims 1-15, wherein the unique drug information includes an expiration date of the medication contained in the bin (32).

17. The pharmacy prescription order filling system of claim 16, further including the computer system (22) alerting a pharmacy worker if the medication contained in the bin (32) is on the verge of exceeding the expiration date by a predetermined amount of time.

## Patentansprüche

1. Ein System zum Einlösen von Rezeptbestellungen für Apotheken, das Folgendes umfasst:
eine automatische Rezepteinlösmaschine (30), die sich an einem ersten Ort befindet und die einzelne Rezeptbestellungen aus mindestens einem Behälter (32) ausgibt, der einen Vorrat an Medikamenten enthält;
den mindestens einen Behälter (32), der funktionsfähig an einem computerlesbaren Etikett (50) in Kommunikation mit einem Computersystem (22) angebracht ist, wobei das computerlesbare Etikett (50) den Behälter (32) für das Computersystem (22) identifiziert;
wobei das Computersystem (22) eine Datenbank enthält, die mindestens einen eindeutigen Arzneimittelidentifikator (UDI) für den in dem Behälter (32) enthaltenen Medikamentenvorrat enthält;
der Behälter (32) mit dem Medikamentenvorrat gefüllt und an einem zweiten, von dem ersten Ort entfernten Ort versiegelt wird;
der Behälter (32) vom zweiten Ort zum ersten Ort transportiert und in die automatische Rezepteinlösmaschine mit dem computerlesbaren Etikett (50) in Kommunikation mit dem Computersystem (22) eingesetzt wird;
wobei das Computersystem (22) ein Etikettlesegerät (52) verwendet, um die auf dem computerlesbaren Etikett (50) enthaltenen Informationen automatisch zu lesen und die UDI automatisch dem Behälter zuzuordnen und die korrekte Platzierung des Behälters (32) in der automatischen Rezepteinlösmaschine (30) zu überprüfen;
einen Vorratsbehälter (33), der ein erstes maschinenlesbares Etikett (50a) enthält, das von dem mit dem Computersystem (22) kommunizierenden Etikettleser (52) lesbar ist, wobei das erste Etikett (50a) UDI-Informationen über in dem Vorratsbehälter (33) enthaltene Medikamente enthält, einschließlich des verbleibenden Volumens oder der Anzahl der in dem Vorratsbehälter (33) enthaltenen Pillen, wobei der Vorratsbehälter (33) aus dem Behälter (32) oder einem separaten Behälter stammt;
die automatische Rezepteinlösmaschine (30), die einen Rezeptbehälter (14) füllt, wobei der Rezeptbehälter (14) ein zweites maschinenlesbares Etikett (50b) aufweist, das daran befestigt ist; und
das Computersystem (22), wenn Medikamente aus dem Vorratsbehälter (33) in den Rezeptbehälter (14) übertragen werden, das Übertragen erkennt und als Reaktion auf das Erkennen des Übertragens zumindest einen Teil der UDI-Informationen von dem ersten Etikett (50a) auf das zweite Etikett (50b) überträgt
wobei die UDI-Informationen mit dem Rezeptbehälter (14) zu einem Lagerbereich (55) wandern, bis der Rezeptbehälter (14) an einen Kunden oder Patienten ausgegeben wird;
das Computersystem (22) bei der Rückgabe des Rezeptbehälters (14) das zweite Etikett (50b), das mit dem zurückgegebenen Rezeptbehälter (14) verbunden ist, und das erste Etikett (50a) des Vorratsbehälters (33) liest und die UDIs des ersten und zweiten Etiketts vergleicht;
wenn die UDIs identisch sind, einschließlich der Chargennummer und des Verfallsdatums sowie des nationalen Arzneimittelcodes, kann der Inhalt des zurückgegebenen Rezeptbehälters (14) wieder in den Vorratsbehälter (33) gefüllt werden; und
wenn die UDIs nicht übereinstimmen, kann der zurückgegebene Rezeptbehälter (14) im Lagerbereich (55) verbleiben, um für eine neue Rezeptbestellung verwendet zu werden.

2. System zum Einlösen von Rezeptbestellungen für die Apotheke nach Anspruch 1, das ferner ein automatisches Verifizierungssystem (60) umfasst, das funktionsfähig mit der automatischen Rezepteinlösmaschine (30) verbunden ist, um die Rezeptbestellung vor der Ausgabe automatisch zu überprüfen.

3. System zum Einlösen von Rezeptbestellungen für die Apotheke nach Anspruch 1, wobei die eingelöste Rezeptbestellung an einen Kunden oder Patienten am ersten Ort ausgegeben wird.

4. System zum Einlösen von Rezeptbestellungen für die Apotheke nach Anspruch 3, wobei der erste Ort eine lokale Apotheke ist und der zweite Ort aus der Gruppe ausgewählt ist, die aus einem Medikamentengroßhändler, einer externen zentralisierten Nachfülleinrichtung, die speziell zum Kennzeichnen und Nachfüllen von Behältern ausgelegt ist, und einem Medikamentenhersteller besteht.

5. System zum Einlösen von Rezeptbestellungen für die Apotheke nach Anspruch 3, wobei die örtliche Apotheke (10) eine Einzelhandelsapotheke ist.

6. System zum Einlösen von Rezeptbestellungen für die Apotheke nach Anspruch 3, wobei die lokale Apotheke (10) eine Apotheke innerhalb einer Gesundheitseinrichtung ist.

7. System zum Einlösen von Rezeptbestellungen für die Apotheke nach Anspruch 1, wobei die eingelöste Rezeptbestellung an einen Kunden oder Patienten an einem dritten Ort ausgegeben wird.

8. System zum Einlösen von Rezeptbestellungen für die Apotheke nach Anspruch 7, wobei der erste Ort eine zentrale Abfüllapotheke (12) ist, der zweite Ort ein Medikamentengroßhändler, eine externe zentrale Nachfülleinrichtung, die speziell für die Kennzeichnung und das Nachfüllen von Behältern ausgelegt ist, oder ein Medikamentenhersteller ist und der dritte Ort eine Einzelhandelsapotheke, eine Apotheke innerhalb einer Gesundheitseinrichtung oder die Adresse eines Kunden ist.

9. System zum Einlösen von Rezeptbestellungen für die Apotheke nach Anspruch 8, wobei der dritte Ort die Adresse eines Kunden ist und die eingelöste Rezeptbestellung vom ersten Ort an die Adresse des Kunden versandt wird.

10. System zum Einlösen von Rezeptbestellungen für die Apotheke nach Anspruch 1, wobei jedes der ersten und zweiten maschinenlesbaren Etiketten ein Radiofrequenz-Identifikations- ("RFID"-) Etikett ist, und ferner ein erstes Etikettenlesegerät (52) in Kommunikation mit dem Computersystem (22), das an dem ersten Ort positioniert ist, und ein zweites Etikettenlesegerät in Kommunikation mit dem Computersystem, das an dem zweiten Ort positioniert ist, umfasst.

11. System zum Einlösen von Rezeptbestellungen für die Apotheke nach Anspruch 10, das ferner eine Vielzahl von ersten Tag-Lesegeräten umfasst, die an dem ersten Ort voneinander beabstandet sind.

12. System zum Einlösen von Rezeptbestellungen für die Apotheke nach Anspruch 1, wobei
der Behälter (32) am zweiten Ort versiegelt wird und von einem zertifizierten Apothekenmitarbeiter inspiziert wird, der den Inhalt am zweiten Ort überprüft,
wobei das System ferner ein drittes maschinenlesbares Etikett umfasst, das funktionsfähig an dem zertifizierten Apothekenmitarbeiter befestigt ist, und das Computersystem (22) in Kommunikation mit dem dritten Etikett steht, um den Standort des zertifizierten Apothekenmitarbeiters relativ zu dem Behälter während der Befüllung und Überprüfung des Behälters zu verfolgen und zu überwachen.

13. System zum Einlösen von Rezeptbestellungen für die Apotheke nach Anspruch 1, wobei der Verschluss des Behälters ein hermetischer Verschluss ist.

14. System zum Einlösen von Rezeptbestellungen für die Apotheke nach Anspruch 1, das ferner einen mit dem Computersystem (22) kommunizierenden Wandler enthält, der aktiviert wird, um einen Apothekenmitarbeiter zu alarmieren, wenn das Computersystem eine vorbestimmte Abweichung im Zusammenhang mit dem Einlösen des individuellen Rezepts feststellt.

15. System zum Einlösen von Rezeptbestellungen für die Apotheke nach Anspruch 14, wobei die vorbestimmte Abweichung aus der Gruppe ausgewählt wird, die aus einer nicht ordnungsgemäßen Medikamentenabgabe aus der automatischen Rezepteinlösmaschine (30), einem nicht ordnungsgemäßen Vorratsbehälter, der in der automatischen Rezepteinlösmaschine positioniert ist, und einer nicht ordnungsgemäßen Entnahme eines ausgefüllten Rezepts aus einem Lagerbereich besteht.

16. System zum Einlösen von Rezeptbestellungen für die Apotheke nach einem der Ansprüche 1 bis 15, wobei die eindeutige Arzneimittelinformation ein Verfallsdatum des in dem Behälter (32) enthaltenen Medikaments enthält.

17. System zum Einlösen von Rezeptbestellungen für die Apotheke nach Anspruch 16, wobei das Computersystem (22) einen Apothekenmitarbeiter alarmiert, wenn das in dem Behälter (32) enthaltene Medikament kurz davor ist, das Verfallsdatum um eine vorbestimmte Zeitspanne zu überschreiten.

## Revendications

1. Un système d'encaissement des commandes d'ordonnances pour les pharmacies comprenant les éléments suivants
une machine automatique d'encaissement d'ordonnances (30), située à un premier endroit, pour dépenser des commandes d'ordonnances individuelles à partir d'au moins un récipient (32) contenant une réserve de médicaments ;
le au moins un récipient (32) attaché de manière opérationnelle à une étiquette lisible par ordinateur (50) en communication avec un système informatique (22), dans lequel l'étiquette lisible par ordinateur (50) identifie le récipient (32) pour le système informatique (22) ;
dans lequel le système informatique (22) comprend une base de données contenant au moins un identifiant unique de médicament (UDI) pour la réserve de médicament contenue dans le récipient (32) ;
le récipient (32) est rempli de la réserve de médicament et scellé à un deuxième endroit éloigné du premier endroit ;
le récipient (32) est transporté du deuxième endroit au premier endroit et est inséré dans la machine automatique d'encaissement d'ordonnances avec l'étiquette lisible par ordinateur (50) en communication avec le système informatique (22) ;
dans lequel le système informatique (22) utilise un lecteur d'étiquettes (52) pour lire automatiquement les informations contenues sur l'étiquette lisible par ordinateur (50) et associer automatiquement l'UDI au récipient et vérifier le placement correct du récipient (32) dans la machine automatique d'encaissement d'ordonnances (30) ;
un récipient de stockage (33) contenant une première étiquette lisible par machine (50a) lisible par le lecteur d'étiquette (52) communiquant avec le système informatique (22), la première étiquette (50a) contenant des informations UDI sur des médicaments contenus dans le récipient de stockage (33), y compris le volume restant ou le nombre de pilules contenues dans le récipient de stockage (33), le récipient de stockage (33) provenant du récipient (32) ou d'un récipient séparé ;
la machine automatique d'encaissement d'ordonnances (30) qui remplit un récipient de prescription (14), le récipient de prescription (14) ayant une deuxième étiquette lisible par machine (50b) fixée à celui-ci ; et
le système informatique (22), lorsque des médicaments sont transférés du récipient de stockage (33) dans le récipient de prescription (14), détecte le transfert et, en réponse à la détection du transfert, transfère au moins une partie des informations UDI de la première étiquette (50a) à la deuxième étiquette (50b)
dans lequel les informations UDI se déplacent avec le récipient de prescription (14) vers une zone de stockage (55) jusqu'à ce que le récipient de prescription (14) soit délivré à un client ou un patient ;
le système informatique (22), lors du retour du récipient de prescription (14), lit la deuxième étiquette (50b) associée au récipient de prescription (14) retourné et la première étiquette (50a) du récipient de stockage (33) et compare les UDI de la première et de la deuxième étiquette ;
si les UDI sont identiques, y compris le numéro de lot et la date de péremption ainsi que le code national du médicament, le contenu du récipient de prescription (14) retourné peut être rempli à nouveau dans le récipient de stockage (33) ; et
si les UDI ne correspondent pas, le récipient de prescription (14) retourné peut rester dans la zone de stockage (55) afin d'être utilisé pour une nouvelle commande de prescription.

2. Système d'encaissement des commandes d'ordonnances pour la pharmacie selon la revendication 1, comprenant en outre un système de vérification automatique (60) de manière opérationnelle à la machine automatique d'encaissement d'ordonnances (30) pour vérifier automatiquement la commande d'ordonnances avant sa délivrance.

3. Système d'encaissement des commandes d'ordonnances pour la pharmacie selon la revendication 1, dans lequel la commande d'ordonnances encaissée est délivrée à un client ou à un patient au premier endroit.

4. Système d'encaissement des commandes d'ordonnances pour la pharmacie selon la revendication 3, dans lequel le premier endroit est une pharmacie locale et le deuxième endroit est choisi dans le groupe constitué par un grossiste en médicaments, une installation de réapprovisionnement centralisée externe spécialement conçue pour marquer et réapprovisionner les récipients et un fabricant de médicaments.

5. Système d'encaissement des commandes d'ordonnances pour la pharmacie selon la revendication 3, dans lequel la pharmacie locale (10) est une pharmacie de détail.

6. Système d'encaissement des commandes d'ordonnances pour la pharmacie selon la revendication 3, dans lequel la pharmacie locale (10) est une pharmacie au sein d'un établissement de santé.

7. Système d'encaissement des commandes d'ordonnances pour la pharmacie selon la revendication 1, dans lequel la commande d'ordonnances encaissée est délivrée à un client ou à un patient dans un troisième endroit.

8. Système d'encaissement des commandes d'ordonnances pour la pharmacie selon la revendication 7, dans lequel le premier endroit est une pharmacie centrale de remplissage (12), le deuxième endroit est un grossiste en médicaments, une installation centrale externe de remplissage spécialement conçue pour l'étiquetage et le remplissage de récipients ou un fabricant de médicaments, et le troisième endroit est une pharmacie de détail, une pharmacie au sein d'un établissement de santé ou l'adresse d'un client.

9. Système d'encaissement des commandes d'ordonnances pour la pharmacie selon la revendication 8, dans lequel le troisième endroit est l'adresse d'un client et la commande d'ordonnances encaissée est envoyée du premier endroit à l'adresse du client.

10. Système d'encaissement des commandes d'ordonnances pour la pharmacie selon la revendication 1, dans lequel chacune des première et deuxième étiquettes lisibles par machine est une étiquette d'identification par radiofréquence ("RFID"), et comprend en outre un premier lecteur d'étiquettes (52) en communication avec le système informatique (22) positionné au premier endroit et un deuxième lecteur d'étiquettes en communication avec le système informatique positionné au deuxième endroit.

11. Système d'encaissement des commandes d'ordonnances pour la pharmacie selon la revendication 10, comprenant en outre une pluralité de premiers lecteurs de "tag" espacés les uns des autres au niveau du premier endroit.

12. Système d'encaissement des commandes d'ordonnances pour la pharmacie selon la revendication 1, dans lequel
le récipient (32) est scellé au deuxième endroit et est inspecté par un préparateur en pharmacie certifié qui vérifie le contenu au deuxième endroit,
dans lequel le système comprend en outre une troisième étiquette lisible par machine fixée de manière opérationnelle au préparateur en pharmacie certifié, et le système informatique (22) est en communication avec la troisième étiquette pour suivre et surveiller le endroit du préparateur en pharmacie certifié par rapport au récipient pendant le remplissage et l'inspection du récipient.

13. Système d'encaissement des commandes d'ordonnances pour la pharmacie selon la revendication 1, dans lequel la fermeture du récipient est une fermeture hermétique.

14. Système d'encaissement des commandes d'ordonnances pour la pharmacie selon la revendication 1, comprenant en outre un transducteur communiquant avec le système informatique (22) et activé pour alerter un employé de la pharmacie lorsque le système informatique détecte une anomalie prédéterminée liée à l'encaissement de l'ordonnance individuelle.

15. Système d'encaissement des commandes d'ordonnances pour la pharmacie selon la revendication 14, dans lequel l'anomalie prédéterminée est sélectionnée dans le groupe constitué par une délivrance non conforme de médicaments à partir de la machine automatique d'encaissement d'ordonnances (30), un récipient de stockage non conforme positionné dans la machine automatique d'encaissement d'ordonnances et un retrait non conforme d'une ordonnance remplie à partir d'une zone de stockage.

16. Système d'encaissement des commandes d'ordonnances pour la pharmacie selon l'une quelconque des revendications 1 à 15, dans lequel l'information unique sur le médicament comprend une date de péremption du médicament contenu dans le récipient (32).

17. Système d'encaissement des commandes d'ordonnances pour la pharmacie selon la revendication 16, dans lequel le système informatique (22) alerte un employé de la pharmacie lorsque le médicament contenu dans le récipient (32) est sur le point de dépasser la date de péremption d'une durée prédéterminée.
